# EUROPEAN PATENT SPECIFICATION

(11) **EP 1 249 246 B1**
(45) Date of publication and mention of the grant of the patent: **28.09.2005**
(21) Application number: 01108923.2
(22) Date of filing: 10.04.2001
(51) Int. Cl.: A61K 31/19, A61K 31/165, A61P 35/00

(54) **Use of an histone deacetylase inhibitor for the treatment of diseases associated with an hpv infection**
Verwendung von Histonedeacetylasehemmern zur Behandlung von Papillomaviren assoziierte Krankheiten
Utilisation des inhibiteurs d'histone déacetylase pour le traitement des maladies associées au papillomavirus

(43) Date of publication of application: 16.10.2002
(73) Proprietor: Deutsches Krebsforschungszentrum Stiftung des öffentlichen Rechts, 69120 Heidelberg (DE)
(72) Inventor: Finzer, Patrick, 68165 Mannheim (DE); Zur Hausen, Harald, 69483 Waldmichelbach (DE); Rösl, Frank, 67141 Neuhofen (DE)
(74) Representative: Schüssler, Andrea

(56) References cited:
- BOFFA L C ET AL: "SUPPRESSION OF HISTONE DEACETYLATION IN-VIVO AND IN-VITRO BY SODIUM BUTYRATE" JOURNAL OF BIOLOGICAL CHEMISTRY, vol. 253, no. 10, 1978, pages 3364-3366, XP001013505 EN ISSN: 0021-9258
- HAGOPIAN H K ET AL: "EFFECT OF N BUTYRATE ON DNA SYNTHESIS IN CHICK FIBROBLASTS AND HELA CELLS" CELL, vol. 12, no. 3, 1977, pages 855-860, XP001013292 EN ISSN: 0092-8674
- CLARK PAUL R ET AL: "A novel drug screening assay for papillomavirus specific antiviral activity." ANTIVIRAL RESEARCH, vol. 37, no. 2, February 1998 (1998-02), pages 97-106, XP002110672 ISSN: 0166-3542
- LIU YONGMIN ET AL: "Human papillomavirus type 16 E6 and HPV-16 E6/E7 sensitize human keratinocytes to apoptosis induced by chemotherapeutic agents: Roles of p53 and caspase activation." JOURNAL OF CELLULAR BIOCHEMISTRY, vol. 78, no. 2, May 2000 (2000-05), pages 334-349, XP002176729 ISSN: 0730-2312
- ZHAO WEI ET AL: "Trichostatin A up-regulates human papillomavirus type 11 upstream regulatory region-E6 promoter activity in undifferentiated primary human keratinocytes." JOURNAL OF VIROLOGY, vol. 73, no. 6, June 1999 (1999-06), pages 5026-5033, XP002176730 ISSN: 0022-538X
- PARK JONG-SUP ET AL: "Inactivation of interferon regulatory factor-1 tumor suppressor protein by HPV E7 oncoprotein: Implication for the E7-mediated immune evasion mechanism in cervical carcinogenesis." JOURNAL OF BIOLOGICAL CHEMISTRY, vol. 275, no. 10, 10 March 2000 (2000-03-10), pages 6764-6769, XP002176731 ISSN: 0021-9258
- LEA MICHAEL A ET AL: "Induction of reporter gene expression by inhibitors of histone deacetylase." ANTICANCER RESEARCH, vol. 18, no. 4A, July 1998 (1998-07), pages 2717-2721, XP001013376 ISSN: 0250-7005
- SAUNTHARARAJAH YOGEN ET AL: "Phenylbutyrate and trichostatin-A induce differentiation of AML1-ETO leukemia cells." BLOOD, vol. 94, no. 10 SUPPL. 1 PART 1, 15 November 1999 (1999-11-15), page 80a XP001013351 Forty-first Annual Meeting of the American Society of Hematology;New Orleans, Louisiana, USA; December 3-7, 1999 ISSN: 0006-4971
- KIM Y.B. ET AL: 'Oxamflatin is a novel antitumour compound that inhibits mammalian histone deacetylase.' ONCOGENE vol. 18, 15 April 1999, pages 2461 - 2470

## Description

The present invention relates to the use of histone deacetylase inhibitors for the treatment of a disease, wherein the disease is cervical cancer, cervical intraepithelial neoplasm, wart, larynx papilloma or condyloma acuminatum associated with a human papilloma virus HPV infection. Particularly useful inhibitors are sodium butyrate, phenylbutyrate and trichostatin A.

Carcinoma of the uterine cervix (cervical cancer, CC) is the second most common cancer in women worldwide and the first in developing countries. CC develops through premalignant intermediate stages of increasing severity known as cervical intraepithelial neoplasm (CIN) grades 1-3, the latter leading to the development of invasive cancer in about 50% of cases over a period of 1-2 decades. More than 11% of the global cancer incidence in women is due to human papillomavirus (HPV) infections. Infection with HPV-types 16 and 18 has been associated with the development of CIN and CC, with HPV genotype 16 being the most prevalent viral type to infect the cervix. The E6 and E7 proteins encoded by these HPV types are thought to be involved in the pathogenesis of CC by inducing abnormal cell proliferation. Expression of E6 and E7 is consistently detected in tissue and tumor cells from HPV-associated CCs. Furthermore, the E6 and E7 genes from HPV types 16 and 18 are sufficient for transformation of epithelial cells in culture.

There is increasing evidence that the E6 and E7 viral oncogenes encoded by HPV types 16 and 18 may be effective targets for tumor rejection by the host and that a therapy might be based on inactivation of said proteins or inhibition of expression of the corresponding genes. Unfortunately, the different strategies employed so far for therapy gave only discouraging results. Theoretically, a treatment might be achieved by methods like, e.g., the suppression of the expression of viral oncogenes using antisense-approaches or interference of specific protein interactions between cellular proteins and viral oncogenes using aptameres. However, at present it is unclear whether these strategies will ever work, since, e.g., as regards antisense-based approaches it is entirely open as to whether in vivo any effect by oncogene suppression can be obtained and with respect to the use of aptamers it is so far entirely unclear as to whether they are even capable of entering an oncogene-positive cell. Moreover both approaches are time and cost consuming, thus, presumably not generally suitable for therapy.

Therefore, it is the object of the present invention to provide means allowing the treatment of diseases associated with an HPV infection wherein the disease is cervical cancer, cervical intraepithelial neoplasm, wart, larynx papilloma, or condyloma acuminatum.

According to the present invention this is achieved by the subject matters defined in the claims. It has been found during the experiments leading to the present invention that the application of histone deacetylase inhibitors results in the functional inactivation of the viral oncogenes of human papilloviruses (HPV) by reactivation of defense mechanisms of the host cell, leading to growth inhibition and induction of apoptosis of the treated cells. In the experiments it could be shown that the histone deacetylase inhibitors sodium butyrate and trichostatin A arrest human papillomavirus (HPV)-positive cells in G1 to S transition of the cell cycle, which is paralleled by an up-regulation of the cyclin dependent kinase inhibitors (CKIs) p21^{CIP1} and p27^{KIP1}. While these CKls normally cannot exert their cdk2-inhibitory function in the presence of the viral oncoprotein E7, co-immunoprecipitation experiments revealed that with binding of p21^{CIP1} and p27^{KIP1} to the cyclin-cdk2 complex, E7 binding is prevented. Although HPV expression is thought to be required to maintain a proliferative phenotype of cervical carcinoma cells, exclusion of E7 and complete suppression of cdk2 activity is achieved even in the presence of ongoing viral transcription. Increase of p27^{KIP1} correlates with down-regulation of p45^{SKP2}, a component of the ubiquitin-protein ligase SCF^{SKP2} which controls the half-life of regulatory proteins during the cell cycle. Besides additional modulatory effects on cyclin expression (cyclin D1 and cyclin A suppression, cyclin E induction), inhibition of histone deacetylation also triggered Rb degradation, while the levels of E2F remained unaffected. The presence of free intracellular E2F and the concomitant induction of p21 and p27 during G1 arrest apparently results in a "conflicting growth situation", which finally renders the cells to undergo apoptosis. In conclusion, the finding that inhibition of histone deacetylation can bypass the transforming potential of "high risk" HPV oncoproteins by inducing a block in G1 to S transition and subsequent apoptosis may have important implications for the treatment of HPV mediated diseases like cervical cancer. Since the HDAC inhibitors used in the examples, below, are not toxic for normal cells, it is apparent that these inhibitors are useful for the treatment of diseases associated with an HPV infection.

Accordingly, the present invention relates to the use of a histone deacetylase inhibitor for the preparation of a medicament for the treatment of a disease associated with an HPV infection, wherein the disease is cervical cancer, cervical intraepithelial neoplasm, wart, larynx papilloma or condyloma acuminatum.

As used herein, the term "histone deacetylase inhibitor" relates to any compound which is capable of inhibiting the activity of histone deacetylase. The person skilled in the art can select suitable compounds on the basis of the known structures (and amino acid sequences) of histone deacetylases, e.g. histone deacetylases 1, 2, 3, 4, 5, 6, 7, 7A, isoform a, 7B, isoform b and 8; see NCBI-Databases AAH00301, XP004370, AAH00614, NP006028, NP005465, NP006035, AAF63491, NP056216, NP057680 and NP060956. Examples of such compounds are antibodies, preferably monoclonal antibodies that specifically react with the histone deacetylase. More recently, WO 98/25146 described further methods for screening libraries of complexes for compounds having a desired property, especially, the capacity to agonize, bind to, or antagonize a polypeptide. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with histone deacetylase inhibitors are, for example, the in vitro screening with the phage display system as well as filter binding assays or "real time" measuring of interaction. All these methods can be used to identify inhibitors of histone deacetylases.

Various sources for the basic structure of such an inhibitor can be employed and comprise, for example, mimetic analogs of the histone deacetylase. Mimetic analogs or biologically active fragments thereof can be generated by, for example, substituting the amino acids that are expected to be essential for the biological activity with, e.g., stereoisomers, i.e. D-amino acids; see e.g., Tsukida, J. Med. Chem. 40 (1997), 3534-3541. Furthermore, in case fragments are used for the design of biologically active analogs pro-mimetic components can be incorporated into a peptide to reestablish at least some of the conformational properties that may have been lost upon removal of part of the original polypeptide; see, e.g., Nachman, Regul. Pept. 57 (1995), 359-370. Furthermore, the histone deacetylase can be used to identify synthetic chemical peptide mimetics that bind to or can function as a ligand, substrate or binding partner of the histone deacetylase as effectively as does the natural polypeptide; see, e.g., Engleman, J. Clin. Invest. 99 (1997), 2284-2292. For example, folding simulations and computer redesign of structural motifs of the histone deacetylase can be performed using appropriate computer programs (Olszewski, Proteins 25 (1996), 286-299; Hoffman, Comput. Appl. Biosci. 11 (1995), 675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of the histone deacetylase and its ligand or other interacting proteins by computer assistant searches for complementary peptide sequences (Fassina, Immunomethods 5 (1994), 114-120). Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptide mimetics of a histone deacetylase. Such pseudopeptide analogues of the natural amino acid sequence of the protein may very efficiently mimic the parent protein (Benkirane, J. Biol. Chem. 271 (1996), 33218-33224). For example, incorporation of easily available achiral ω-amino acid residues into a histone deacetylase results in the substitution of amide bonds by polymethylene units of an aliphatic chain, thereby providing a convenient strategy for constructing a peptide mimetic (Banerjee, Biopolymers 39 (1996), 769-777). Appropriate peptide mimetics of histone deacetylases can also be identified by the synthesis of peptide mimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, a three-dimensional and/or crystallographic structure of a histone deacetylase can be used for the design of peptide mimetic inhibitors (Rose, Biochemistry 35 (1996), 12933-12944; Rutenber, Bioorg. Med. Chem. 4 (1996), 1545-1558).

Preferred histone deacetylase inhibitors are sodium butyrate, phenylbutyrate and trichostatin A, respectively. Particularly preferred are derivatives of said inhibitors showing increased pharmalogical half-life (Brettman and Chaturvedi, J. Cli. Pharmacol. 36 (1996),617-622).

The present invention particularly, but not exclusively, relates to the use of a histone deacetylase inhibitor for the preparation of a medicament for the treatment of a disease associated with an infection with an HPV of the HPV-16 and HPV-18 genotypes and wherein the disease is cervical cancer, cervical intraepithelial neoplasm, wart, larynx papilloma or condyloma acuminatum. It will be, however, appreciated that the invention extends to variants of such HPV genotypes and other HPV genotypes, e.g. HPV1 or HPV11, which may have oncogenic or other pathologic potential.

For administration these histone deacetylase inhibitors are preferably combined with suitable pharmaceutical carriers. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the disease, general health and other drugs being administered concurrently.

### Brief description of the drawings

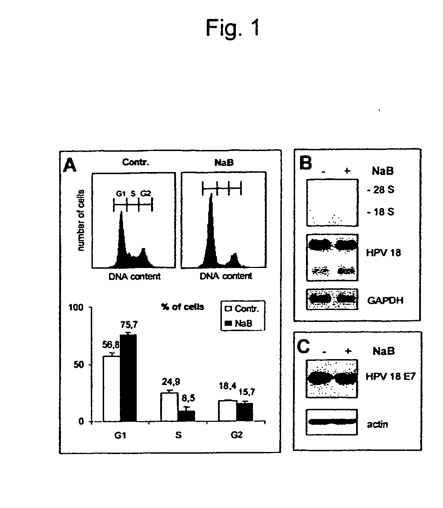

### Figure 1: Sodium butyrate (NaB) inhibits G1 to S phase transition in HeLa cells without affecting viral oncogene expression

(A) Upper part: representative flow-cytometric profile of HeLa cells after treatment with 6 mM sodium butyrate (NaB) for 16 hours. "Contr.": untreated control. The different phases of the cell cycle are indicated (G1, S, G2, respectively). Lower part: quantification of the number of cells during the cell cycle (means of three independent experiments).
(B) Northem blot analysis. 5 µg of RNA were separated in a 1% agarose gel. The filter was consecutively hybridized with a HPV18 and GAPDH probe. The positions of the 28S and 18S ribosomal RNA are indicated.
(C) Western blot analysis. 75 µg of total cellular protein was loaded on a 12% SDS-PAGE gel. After electrotransfer, the filters were consecutively incubated with HPV18 E7 and a monoclonal actin antibody. (-) : untreated cells; (+): treated with 6 mM sodium butyrate for 16 hours.

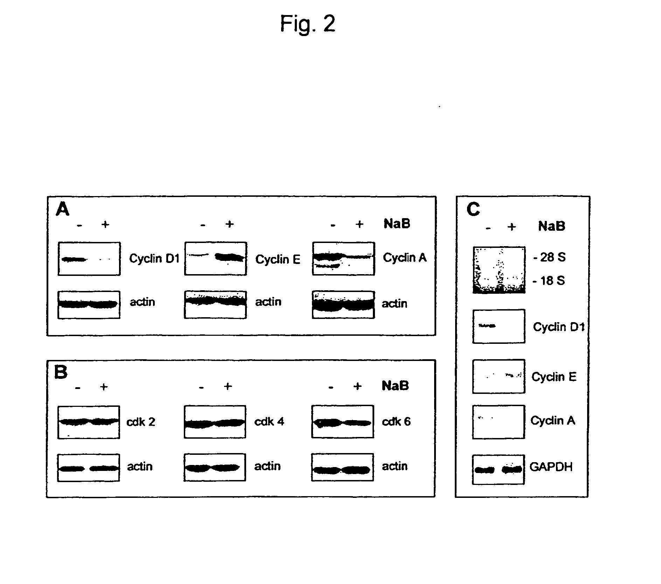

### Figure 2: Effects of sodium butyrate on the expression of G1 cyclins and cyclin dependent kinases (cdks)

(A and B) Exponentially growing HeLa cells were treated with sodium butyrate (NaB) as indicated in Fig. 1. 50 µg of protein were separated on 12% SDS-PAGE minigels. After electrotransfer, the filters were incubated with antibodies against cyclin D1, cyclin E, cyclin A or cdk2, cdk4 or cdk6 specific antibodies. Equal protein loading was confirmed by reincubating the filters with a monoclonal actin antibody.
(C) Northem blot analysis. 5 µg of RNA were separated in a 1% agarose gel. The filters were hybridized with cDNAs encoding cyclin D1, cyclin E, cyclin A and GAPDH. The positions of the 28S and 18S ribosomal RNA are indicated. (-): untreated cells; (+): treated with 6 mM sodium butyrate for 16 hours.

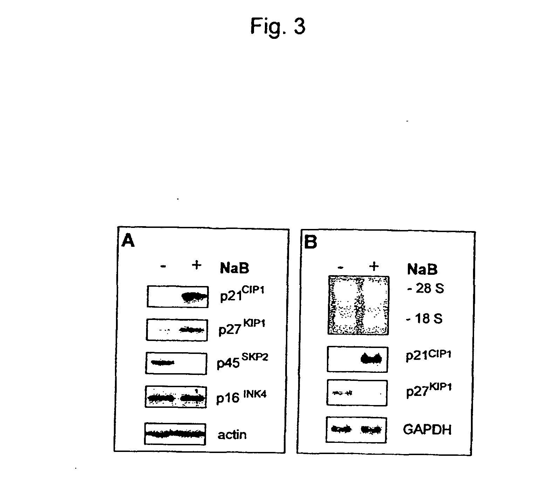

### Figure 3: Regulation of cyclin-dependent kinase inhibitors and suppression of p45^{SKP2} by sodium butyrate

(A) Westem blot analysis. 50 µg of cellular protein were loaded on SDS-PAGE minigels. After electrotransfer, the filters were incubated with p21^{CIP1}, p27^{KIP1}, p16^{INK4} and p45^{SKP2}. Equal protein loading was confirmed by a monoclonal actin antibody.
(B) Northem blot analysis. 5 µg of RNA were separated in a 1% agarose gel. Filters were hybridized with cDNAs encoding p21^{CIP1}, p27^{KIP1} and GAPDH. The positions of the 28S and 18S ribosomal RNA are indicated. (-): untreated cells; (+): treated with 6 mM sodium butyrate for 16 hours.

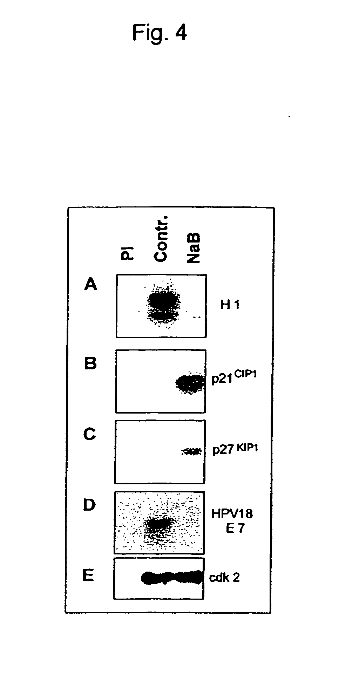

### Figure 4: Sodium butyrate suppresses cdk2 activity by enhanced interaction with p21 and p27 and concomitant loss of E7 binding

(A) Autoradiography. Cdk2 complexes were immunoprecipitated from HeLa cells and assayed for their activity using histone H1 as substrate. ("P1": preimmune serum).
(B-E) Western blot analysis of the cdk2 complex. Cdk2 precipitates were separated in a 12% SDS-PAGE gel and immunoblotted with p21^{CIP1} (B), p27^{KIP1} (C) and HPV18 E7 specific antibodies.
(D) Equal loading was verified by incubation with cdk2 antibodies.
(E). P1: preimmune serum. Contr.: untreated cells; NaB: treated with 6 mM sodium butyrate for 16 hours.

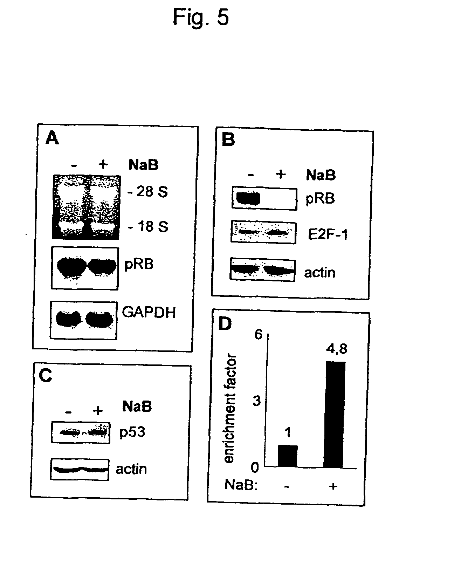

### Figure 5: Sodium butyrate mediates pRb degradation without affecting the level of E2F-1: induction of apoptosis in HeLa cells

(A) Northern blot analysis. The same filter was consecutively hybridized with a pRB and GAPDH-specific cDNA. The positions of the 28S and 18S ribosomal RNA in the ethidium bromide stained agarose gel are indicated.
(B and C) Western blot analysis. 50 µg of protein were separated in 8% (for pRb) and 12% SDS-PAGE gels. After electrotransfer, the filters were incubated with pRb, E2F-1 (B) and (C) p53 specific antibodies. Equal protein loading was confirmed with a monoclonal actin antibody.
(D) Quantification of apoptosis using a commercially available "Cell Death Detection ELISA" kit. The enrichment factor, for untreated control cells arbitrarily set as 1, directly reflects the extent of apoptosis in HeLa cells after treatment with sodium butyrate for 16 hours.

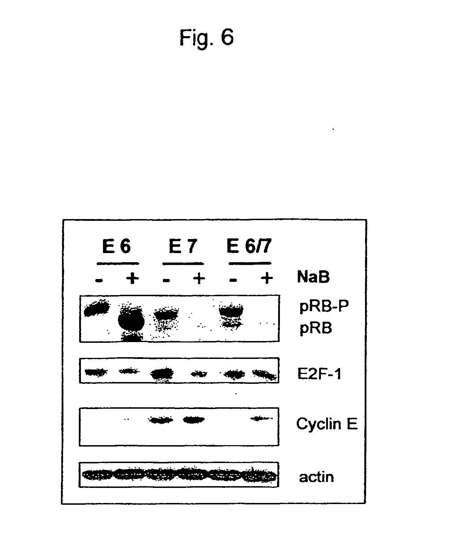

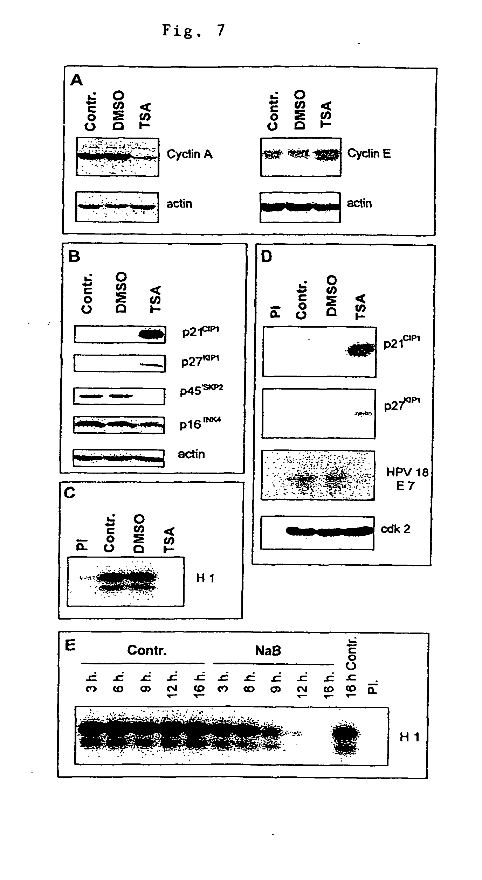

### Figure 6: pRB degradation by sodium butyrate in HPV16 immortalized keratinocytes is E7 dependent

Western blot analysis of cellular extracts obtained from E6-, E7-, and E6/E7-Immortalized cells which were separated in 8% (pRb) and 12% SDS-PAGE gels. After electrotransfer, the filters were incubated with pRb, E2F-1, cyclin E and actin antibodies. ( - ): untreated cells; ( + ): treated with 6 mM sodium butyrate for 16 hours. Due to quantitative differences of pRb and cyclin E levels in E7- and E6/E7-positive cells, the filters were exposed for different times.

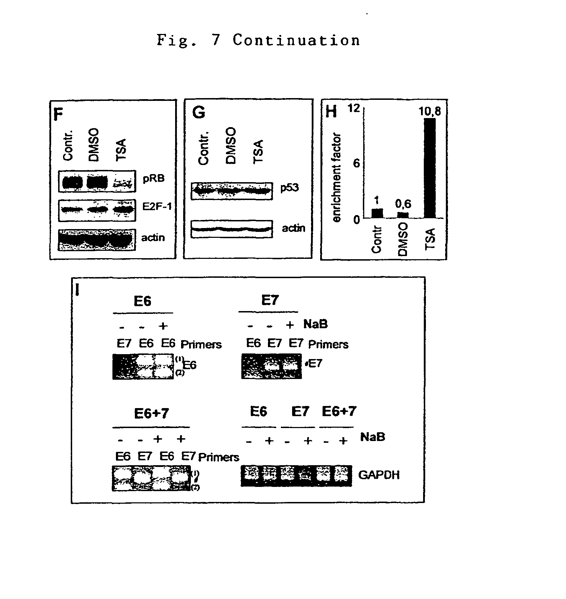

### Figure 7: Effects of trichostatin A on the expression of cyclins and cyclin-dependent kinase inhibitors and the pRb degradation

The present invention is explained by the examples.

### Example 1

### General methods

### (A) Cell lines

HPV18-positive cervical Carcinoma cells (HeLa) were maintained in Dulbecco's modified Eagle's medium (DMEM), supplemented with 10 % fetal calf serum (Gibco BRL, Rockville, USA), 1% penicillin and streptomycin (Sigma, Deisenhofen, Deutschland). Primary human keratinocytes, immortalized by E6-, E7- and E6/E7-open reading frames carrying amphotroptic retroviruses were cultivated in "Keratinocyte Medium Kit" (Sigma).

### (B) Reagents

The sodium salt of n-butyric acid (Sigma) was freshly resolved and diluted with cultivation medium. Trichostatin A (Sigma) was prepared in dimethylsulfoxid (DMSO) (Merck, Darmstadt, Deutschland).

### (C) Cell cycle analysis

Cells were harvested by trypsinisation, washed twice with phosphate-buffered saline (PBS) and fixed overnight with 70 % ethanol. The cells were resuspended in PBS containing 40 µg/ml of DNase-free RNase A and 50 µg/ml propidium iodide and cell cycle distribution was measured in a fluorescence-activated cell sorter (FACSort) from Becton Dickinson, San Jose, USA. DNA content was quantified by using the "Cell Quest" software (Becton Dickinson, San Jose, USA).

### (D) RNA extraction and Northern blot analysis

Total cellular RNA was extracted according to the guanidinium-thiocyanate procedure (Chomczynski and Sacchi, Anal. Biochem. 162 (1987), 156-159). Approximately 5 µg RNA were separated on 1% agarose gels in the presence of ethidium bromide under non-denaturing conditions and transferred to GeneScreen Plus membranes (DuPont, Bad Homburg, Deutschland). The filters were hybridized under stringent conditions with specific probes, which were labeled with ³²p-dCTP by random priming (Feinberg and Vogelstein, Anal. Biochem. 137 (1984), 266-267).

### (E) DNA hybridization probes

The cDNAs of p21 (el-Deiry et al., Cell 75 (1993), 817-825), p27 (Polyak et al., Cell 78 (1994), 59-66), cyclin D1 (Baldin et al., Genes Dev. 7 (1993), 812-821), cyclin A (Pines and Hunter, Nature 346 (1990), 760-763) and cyclin E (Hinds et al., Cell 70 (1992), 993-1006) were used. The cDNA stretch of pRB (nucleotide 379-928) was obtained from M. Tommasino (DKFZ Heidelberg). The GAPDH probe (Ercolani et al., J. Biol. Chem. 263 (1988, 15335-15341) was obtained from A. Alonso (DKFZ, Heidelberg). The unit-length HPV18 genome was cloned in pBR322 (Boshart et al., EMBO J. 3 (1984), 1151-1157).

### (F) SDS-PAGE and Western blots

Cellular extracts were separated in 8-12% SDS PAGE gels and electrotransferred as described by Finzer et al. (Oncogene 19 (2000), 3235-3244). The following antibodies were used. cyclin E (HE 12), cyclin D 1 (HD 11), cdk2 (M2), cdk4 (C-22), cdk6 (C-21), p27^{KIP1} (C-19), p45^{SKP2} (N-19), HPV18-E7 (N-19) and E2F-1 (KH95) (Santa Cruz Biotechnology, Inc. Santa Cruz, USA), p21^{CIP1} (C24420; Transduction Laboratories,Lexington,USA), pRB (NCL-RB; Novocastra, UK) and p16^{INK4} (15126E, Pharmingen,San Diego, USA). Cyclin A was kindly provided by M. Pagano (Pagano et al., EMBO J. 11 (1992), 961-971). Equal protein transfer and loading was routinely checked by incubating the filters with a monoclonal actin antibody (ICN Biomedicals, Ohio, USA).

### (G) Extract preparation, immunoprecipitation and histone kinase assays

For cell fractionation, cell monolayers were washed twice with PBS and harvested by trypsinisation. Cell extract preparation and cdk2 activity assays were exactly done as described by Blomberg and Hoffmann (Mol. Cell. Biol. 19 (1999), 6183-6194). In addition, cdk2 was immunoprecipitated and analyzed by immunoblotting. Beads used for cdk2 kinase assay were washed 3 times with lysis buffer (Blomberg and Hoffmann, 1999), incubated with Laemmli sample buffer and boiled for 5 min. Supernatant was analyzed by SDS-PAGE gels. Immunoblotting was carried out with the following antibodies: p21^{CIP1} (C24420) and p27^{KIP1} (K25020; Transduction Laboratories) or HPV18-E7 (N-19; Santa Cruz, Inc.). Cdk2 specific antibodies or preimmune serum were kindly provided by I. Hoffmann (DKFZ, Heidelberg).

### (H) Quantification of apoptosis

The rate of apoptosis was determined with the Cell Death Detection kit (ELISA^{PLUS} , Roche Diagnostics, Mannheim, Germany) following the instructions of the manufacturer.

### Example 2

### Histone deacetylase (HDAC) inhibitors induce G1/S phase arrest in HPV18-positive cervical carcinoma cells despite ongoing E6/E7 synthesis

Modulation of histone acetylation is an integral part of a regulatory mechanism, which is involved in the nucleosomal organization of the bulk cellular DNA. Posttranslational neutralisation of the positive charge of lysine residues within the N-terminal domain of core histones relieves histone-DNA interactions, which in turn facilitates the accessibility of transcription factors with their cognate regulatory elements at the DNA level. Alterations of the chromatin architecture is mediated by an interplay between histone acetylases (HAT) and deacetylases (HDAC) counteracting each other in either activating or repressing gene expression. During the last years, a substantial number of HATs have been characterized in yeast and Tetrahymena. In higher eukaryotes, particular adaptor molecules such as p300/CBP or p/CAF (termed as p300/CPB-associated factor) possess intrinsic HAT activity. Their association with CREB, c-jun, c-fos or unliganded nuclear receptors provides a functional linkage between transcriptional co-activators and histone acetylators during initiation of gene expression. Conversely, histone deacetylase type 1 (HDAC1) is an inherent component of a general corepressor complex which interacts with YY-1, Mad/Max as well as the retinoblastoma protein pRb, regularly leading to inhibition of gene expression, although exceptions exist (Workman and Kingston, Annu. Rev. Biochemn. 67 (1998), 545-579; Wade et al., Trends Biochem. Sci. 22 (1997), 128-132). Since both HATs and HDACs by themselves have no sequence-specifie DNA-binding affinities, physical interaction with transcriptional activators or repressors provides a reliable explanation by which enzymes, normally acting entirely in a global way during nucleosomal remodelling, can be specified to locally defined transcription units.

Not only cellular transcription factors can target HAT and HDAC molecules, but also viral oncoproteins such as E6 and E7 of the "high-risk" human papillomaviruses (HPV), the etiological agents of cervical cancer. As shown recently, HPV16 E6 is capable of abrogating the costimulatory function of CBP and p300, resulting in a decreased ability of these factors to trans-activate p53-, NF-KB- and c-jun-responsive promoter elements. While E6 interferes with the CBP/p300 tethering function to other transcription factors and possibly with intrinsic HAT activity, E7 oncoprotein can indirectly bind to the histone deacetylase complex via the bridge-protein Mi2ß. This property presumably enables E7 to inactivate cellular genes incompatible with the outgrowth of premalignant cells during development of cervical cancer. For example, the interferon-regulatory factor-1 (IRF-1) gene, whose expression is important for interferon signaling and immunological surveillance of persisting HPV infections, is silenced via an E7-mediated recruitment of HDAC to the respective promoter. Depending on the interplay between different transcription factors, E7 can also act in an opposite way. For example, E7 relieves the repressive effect of pRb and HDAC1 on the cyclin E promoter thereby promoting unscheduled cell cycle progression.

Another hallmark of HPV-induced transformation is the post-translational interaction of E6 and E7 with cellular proteins engaged in cell cycle control: E6 binds to p53 and promotes its degradation via the ubiquitin/proteosome pathway. E7 complexes with the retinoblastoma protein pRb, p107, p130, cyclin A, cyclin E as well as the cyclin-dependent kinase inhibitors p21^{CIP1} and p27^{KIP1}.

In order to elucidate the molecular effects of histone deacetylase inhibition in the context of HPV16/18-induced carcinogenesis, the HPV18-positive cervical carcinoma cell line HeLa as well as primary human foreskin keratinocytes, which were separately immortalized with amphotropic retroviruses carrying the open reading frames of HPV16 E6, E7 or E6/E7 were used in the experiments of the present invention. In order to assess the specific impact of histone deacetylase inhibition on cell proliferation, flow cytometric analysis of cellular DNA content was carried out (Fig. 1). Treatment with 6 mM sodium butyrate for 16 hours resulted in a significant increase of the G1 fraction (from 56.8% to 75.7%), whereas the number of cells in S-phase was diminished (from 24.9% to 8.5%). As previously shown, continuous expression of the viral oncogenes seems to be necessary to maintain the proliferative phenotype of cervical carcinoma cells. To address the question whether HDAC inhibitors also have consequences on the transcriptional activity of endogenous HPV18 genomes in HeLa cells, Northern blot analyses were performed. Although time course experiments reproducibly revealed an initial down-regulation of viral E6/E7 expression shortly after sodium butyrate addition, the effect was only transient. However, under conditions where cells became growth arrested (Fig. 1A), still ongoing HPV18 transcription (Fig. 1B) and E7 oncogene-expression could be discerned (Fig. 1 C). Similar results were obtained with trichostatin A, indicating that HDAC inhibitors in general are able to arrest proliferation of cervical carcinoma cells by circumventing and/or neutralizing viral oncoprotein function.

### EXAMPLE 3

### HDAC inhibitors modulate cyclins but not cdk expression

Because the precise mechanism underlying the growth inhibitory effect on cervical carcinoma cells has not yet been established, the steady-state levels of regulatory proteins involved in cell cycle control were examined. As shown in Fig. 2, sodium butyrate selectively down-regulates cyclin D1 and cyclin A, while the amount of cyclin E was strongly increased (Fig. 2A). Incubation of the same filter with a monoclonal actin antibody confirmed equal loading and protein transfer. Quantitative differences of cyclin expression after sodium butyrate application were presumably not due to altered degradation rates, since there was a good accordance between the levels of protein and the corresponding mRNAs (Fig. 2C). Control hybridization of the identical blot with glyceraldehyde-3-phosphate dehydrogenase (GAPDH) again corroborated the selectivity of this effect. In contrast, neither the cyclin-dependent kinase 2 (cdk2) nor cdk4 or cdk6 revealed any significant influence on their expression rates (Fig. 2B), clearly excluding the possibility that the growth-inhibitory effect could be attributed to a mere reduction of the intracellular amounts of certain cdks.

### Example 4

### Sodium butyrate induces cyclin-dependent kinase inhibitor p21^{CIP1} on transcriptional level, while p27^{KIP1} is up-regulated post-translationally by concomitant suppression of p45^{SKP2}, a component of the ubiquitin-protein ligase SCF^{SKP2}

In a next set of experiments, the levels of cyclin-dependent kinase inhibitors (CKIs), which are known mediators of cell cycle arrest upon various antiproliferative signals were investigated. Western blot analysis of cellular extracts (Fig. 3A) demonstrates that the Cip/Kip family members p21^{CIP1} and p27^{KIP1}, which normally block cyclin A/cdk2 and cyclin E/cdk2 function, were significantly induced after sodium butyrate treatment. In contrast, upon examining the same extracts for the cyclin D1-cdk4/6 inhibitor p16^{INK4}, no reduction was discerned. Surprisingly, monitoring the steady-state levels of the corresponding mRNAs, only p21^{CIP1} was increased, while p27^{KIP1} expression was even diminished under the same experimental conditions. To understand this apparent discrepancy, attention was focused on the expression of p45^{SKP2}, a key regulatory protein recently shown to control the intracellular half-life of p27^{KIP1}. p45^{SKP2} which is found to be up-regulated in many transformed cells, became strongly suppressed after addition of sodium butyrate (Fig. 3A). Since the same results were obtained with trichostatin A, these data strongly imply that at least one major cell growth inhibitory function of histone deacetylase inhibitors can be attributed to the post-translational stabilization of the cdk2 inhibitor p27^{KIP1} via disturbance of the SCF (SKP-1-CDC53-F-box) ubiquitin protein ligase complex.

### Example 5

### Cdk2 activity is completely suppressed after butyrate treatment: exclusion of E7 from the complex

Cdk2 inhibitors such as p21^{CIP1} and p27^{KIP1} play an important role during immortalization and cellular transformation by potential DNA tumorviruses, because their function can be neutralized, or bypassed after binding of viral oncoproteins such as E7. To analyze whether cdk2 activity was modulated after addition of sodium butyrate, cyclin-cdk2 complexes were first immunoprecipitated using specific antibodies directed against cdk2, and subsequently assayed in vitro using histone H1 as substrate (Fig. 4A). In comparison with the untreated control, cdk2 kinase activity was completely abolished 16 hours after sodium butyrate addition. Time course experiments performed in parallel showed that cdk2 was still active after 9 hours, but abruptly declined 3 hours later. To test whether or not p21^{CIP1} and p27^{KIP1} in fact bind to the cdk2 complex, the composition pattern of the cdk2 immunoprecipitates was examined by Western blot analysis. As depicted in Fig. 4B and C, cdk2 activity was abrogated under conditions where both CKls became associated with the cdk2 complex. In contrast, E7 that was readily detectable in untreated control cells, completely disappeared in the treated cells though the total intracellular net amount of the viral oncoprotein did not change (Fig. 4D), see also Fig. 1C, for comparison). Reincubation of the same immunoblots with a cdk2 antibody confirmed that equal amounts were precipitated (Fig. 4E). Taken together, these data indicate that transition from a proliferative to a quiescent phenotype apparently requires at least two steps: functional abolition of cdk2 activity by p21^{CIP1} and p27^{KIP1} as well as concomitant prevention of E7 binding.

### Example 6

### Differential pRb inactivation in the presence of individual oncoproteins: the role of histone deacetylase inhibitors in the induction of apoptosis.

The most studied G1 specific cyclin/cdk substrate is the retinoblastoma protein pRb, which can recruit HDAC1 to repress cell cycle regulatory proteins such as cyclin E. Since cyclin E was significantly up-regulated by sodium butyrate, it was mandatory to examine the fate of pRb in the experimental cell systems. Fig. 5B illustrates that pRB became totally degraded after 16 hours, but histone deacetylase inhibition apparently has no consequences on gene expression and translation of the transcription factor E2F-1. Instead, the absence of pRb could be clearly attributed to a post-translational event, because the steady-state level of the corresponding mRNA was maintained (Fig. 5A). The lack of a modulatory effect on p53 (Fig. 5C), whose half-life is controlled by E6, confirmed the previous finding that viral oncoprotein expression is sustained in the presence of sodium butyrate (see Fig. 1 C).

The strong degradation of the anti-apoptotic protein pRb without any reduction of the transcription factor E2F-1 presumably accounts for the final appearance of the distinct apoptotic figures such as membrane blebbing, detachment from the surface and karyorhexis after longer HDAC inhibition. The start of apoptosis can already be detected after 16 hours using a sensitive ELISA assay, which measures the release of histone-associated-DNA-fragments in the cytoplasm after nuclear breakdown. As shown in Fig. 5D, both sodium butyrate (and much stronger trichostatin A) induce programmed cell death to a significant extent.

While E2F-1 also did not significantly vary in HPV16-immortalized human keratinocytes, pRb degradation seems to be strictly dependent on the presence of E7. As shown in Fig. 6, pRb completely disappeared exclusively in E7-expressing cells, while it is only seen to be hypophosphorylated in cells containing E6 as viral oncogene. E6/E7-immortalized keratinocytes again revealed pRb degradation, clearly showing that the fate of pRb is determined by E7 in a dominant fashion. Biological availability of pRb can be followed by monitoring the expression level of cyclin E, whose transcription is negatively regulated by pRb. The reason for cyclin E expression in untreated E7-positive cells is consistent with the ability of E7 to overcome the pRb suppressive effect on the cognate promoter by destroying the pRb-HDACI complex.

### Example 7

### Effects of trichostatin A on the expression of cyclins and cyclin-dependent kinase inhibitors and pRb degradation

The effects of trichostatin A are shown in Fig. 7 (a) - (i)
**7 (a) Trichostatin A down-regulates cyclin A and up-regulates cyclin E.**
   Exponentially growing HeLa cells were treated with trichostatin A as indicated in Fig. 1. 50µg of protein was separated on 12% SDS-PAGE minigels. After electrotransfer, the filters were incubated with antibodies against cyclin E and cyclin A. Equal protein loading was confirmed by reincubating the filters with a monoclonal actin antibody. (Contr.): untreated cells; (DMSO): DMSO control; (TSA): treated with 330 nM trichostatin A for 16 hours.
**7 (b) Trichostatin A induces cyclin-dependent kinase inhibitors p21**^{**CIP1**}**, up-regulates p27**^{**KIP1**} **and suppresses p45**^{**SKP2**}**.**
   Western blot analysis. 50 µg of cellular protein was loaded on SDS-PAGE minigels. After electrotransfer, the filters were incubated with p21^{CIP1}, p27^{KIP1}, p16^{INK4} and p45^{SKP2}. Equal protein loading was confirmed by a monoclonal actin antibody. (Contr.): untreated cells; (DMSO): DMSO control; (TSA): treated with 330 nM trichostatin A for 16 hours.
**7 (c)**
   **(d) Trichostatin A suppresses cdk2 activity by enhanced interaction with p21**^{**CIP1**} **and p27**^{**KIP1**} **and concomittant loss of E7 binding.**
   (c) Autoradiography: Cdk2 complexes were immunoprecipitated from HeLa cells and assayed for their activity using histone H1 as substrate. ("PI": preimmune serum).
   (d) Western blot analysis of the cdk2 complex. Cdk2 precipitates were separated in a 12% SDS-PAGE gel and immunoblotted with p21^{CIP1}, p27^{KIP1} and HPV 18 E7 specific antibodies. Equal loading was verified by incubation with cdk2 antibodies. PI: preimmune serum. (Contr.): untreated cells; (DMSO): DMSO control; (TSA): treated with 330 nM trichostatin A for 16 hours.
**7 (e) Time course of cdk2 suppression after treatment with sodium butyrate.**
   Autoradiography: cdk2 complexes were immunoprecipitated from HeLa cells and assayed for their activity as in (c). Cells were treated for 3, 6, 9, 12 and 16 hours with 6 mM sodium butyrate (NaB). (Contr.): untreated cells harvested after 3, 6, 9, 12, and 16 hours.
**7 (f)**
   **(g)**
   **(h) Trichostatin A mediates pRb degradation without affecting the level of E2F-1: induction of apoptosis in HeLa cells.**
   (f and g) western blot analysis: 50µg of protein was separated in 8% (for pRb) and 12% SDS-PAGE gels. After electrotransfer, the filters were incubated with pRb, E2F-1 (f) and (g) p53 specific antibodies. Equal protein loading was confirmed with a monoclonal actin antibody.
   (h) Quantification of apoptosis using a commercially available "Cell Death Detection ELISA" kit. The enrichment factor, for untreated control cells arbitrarily set as 1, directly reflects the extent of apoptosis in HeLa cells after treatment with trichostatin A for 16 hours. (Contr.): untreated cells; (DMSO): DMSO control; (TSA): treated with 330nM trichostatin A for 16 hours.
**7 (i) Detection of HPV 16 E6 and E7 mRNAs by RT-PCR in the immortalized keratinocytes.**
   The expression of E6 and E7 was detected after electrophoresis of the reverse transcriptase (RT)-PCR products on 2 % agarose gels. Expected E6, E6* or E7 fragments are indicated. As control GAPDH mRNA was detected by RT-PCR (460 bp). The RT-reaction was performed with the Superscript TMII (Gibco) following the attached instructions of the manufacturer, using 1,5 µg of total RNA. For E6 detection, the following primers were used: upper primer 5' ACT GCA ATG TTT CAG GAC CC 3', lower primer 5' TCA GGA CAC AGT GGC TTT TG 3', for the E7 detection; upper primer 5' CCC AGC TGT AAT CAT GCA TG 3', lower primer 5' TGC CCA TTA ACA GGT CTT CC 3'. For GAPDH detection, the following forward (TGG ATA TTG TTG CCA TCA ATG ACC) and reverse (GAT GGC ATG GAC TGT GGT CAT G) primers were used. Conditions for all sets of primers were: initially denaturation time of 3 min. at 94°C and then 30 sec. at 94°C, 1 min. at 60°C, 1 min. at 72°C (35 cycles) and 10 min. at 72°C. (1): E6 and (2) E6* fragments; (#): E7 fragment; (+): treated with 6 mM sodium butyrate for 16 hours; (-): untreated cells.

## Claims

1. Use of a histone deacetylase inhibitor for the preparation of a medicament for the treatment of a disease associated with an human papillomavirus (HPV) infection, wherein the disease is cervical cancer, cervical intraepithelial neoplasm, wart, larynx papilloma or condyloma acuminatum.

2. Use according to claim 1, wherein the histone deacetylase inhibitor is sodium butyrate, phenylbutyrate or trichostatin A.

## Patentansprüche

1. Verwendung eines Histon-Deacetylase Inhibitors zur Herstellung eines Medikaments für die Behandlung einer Krankheit, die mit einer humanen Papillomavirusinfektion assoziiert ist, wobei die Krankheit Zervixkrebs, zervikales intraepitheliales Neoplasma, Warze, Larynxpapillom oder Condyloma acuminatum ist.

2. Verwendung nach Anspruch 1, wobei der Histon-Deacetylase Inhibitor Natriumbutyrat, Phenylbutyrat oder Trichostatin A ist.

## Revendications

1. Utilisation d'un inhibiteur d'histone désacétylase pour la préparation d'un médicament destiné au traitement d'une maladie associée avec une infection du papillomavirus humain (HPV) dans laquelle la maladie est le cancer du col de l'utérus, le néoplasme intraépithélial du col de l'utérus, la verrue, le papillome du larynx ou le condylome acuminé.

2. Utilisation selon la revendication 1, dans laquelle ledit inhibiteur d'histone désacétylase est le butyrate de sodium, le phénylbutyrate ou le trichostatine A.
